# EUROPÄISCHE PATENTSCHRIFT

(11) **EP 0 877 597 B1**
(45) Veröffentlichungstag und Bekanntmachung des Hinweises auf die Patenterteilung: **18.07.2001**
(21) Anmeldenummer: 97908122.1
(22) Anmeldetag: 17.01.1997
(51) Int. Cl.: A61K 7/50, A61K 7/00

(54) **KOSMETISCHES HAUTREINIGUNGSMITTEL AUF BASIS NATÜRLICHER WIRKSTOFFE**
COSMETIC SKIN CLEANSER BASED ON NATURAL ACTIVE SUBSTANCES
AGENT NETTOYANT COSMETIQUE POUR LA PEAU A BASE DE PRINCIPES ACTIFS NATURELS

(30) Priorität: 17.01.1996 DE 19603019
(43) Veröffentlichungstag der Anmeldung: 18.11.1998
(73) Patentinhaber: LANCASTER GROUP GmbH, 55116 Mainz (DE)
(72) Erfinder: MENZEL, Anette, Morris Plains, NJ 07950 (US); MACCHIO, Ralph, Flanders, NJ 07836 (US); STANZL, Klaus, White Plains, NJ 10605 (US); ZASTROW, Leonhard, MC-98000 Monte-Carlo (MC)
(74) Vertreter: Walter, Wolf-Jürgen
(86) Internationale Anmeldenummer: DE9700117
(87) Internationale Veröffentlichungsnummer: WO9725974

(56) Entgegenhaltungen:
- EP-A- 0 254 447
- WO-A-93/13754
- US-A- 5 215 759

## Beschreibung

Die Erfindung betrifft ein kosmetisches Hautreinigungsmittel auf Basis natürlicher Inhaltsstoffe mit einer besonders milden und schonenden Wirksamkeit auf die Haut.

Aus der US-A-46732526 ist ein Mittel zur Tiefenreinigung der Haut bekannt, das eine wasserfreie Reinigungszusammensetzung darstellt mit einer öligen Phase, einem Emulgiermittel und einer teilchenförmigen Schleifsubstanz wie Xanthangummi, Carboxymethylamide, Celluloseether, Hydroxyalkylcellulose oder Copolymerisate von Acrylsäure und Acrylamid.

Aus der WO94/12151 sind Hautreinigungsmittel auf Basis von agglomerierten Siliciumdioxidteilchen bekannt.

Die CH-A-678488 beschreibt Polyethylen-Mikrokügelchen als Grundlage für eine Reinigungszusammensetzung.

Der vorliegende Erfindung liegt die Aufgabe zugrunde, ein Hautreinigungsmittel zu entwickeln, das als Wirkstoffe im wesentlichen natürliche kosmetisch wirksam Bestandteile enthält und dabei eine milde aber ausreichend intensive Hautreinigung erzielt.

Diese Aufgabe wird erfindungsgemäß gelöst mit einem kosmetisches Hautreinigungsmittel auf Basis von im wesentlichen natürlichen Wirkstoffen, das gekennzeichnet ist durch eine wäßrige, nicht-ölige Suspension, bestehend aus
(a) Mikrokügelchen eines Polyoxymethylenharnstoffes, die in ihrem Inneren ein flüssiges, natürliches, pflanzliches öl tragen,
(b) Mitteln auf natürlicher Basis zur Anlagerung von Wasser an die Haut, ausgewählt unter Aloe vera Gel, Jojoba-Öl, Cetearyl glucoside, Lipacide PVB und Gemischen davon, sowie als weiteren Stoff Propylenglycol,
(c) einem oder mehreren natürlichen Emulgatoren,
(d) natürlichen waschaktiven Substanzen, und
(e) weiteren Hilfs- und Trägerstoffen.

Lipacide PVB ist ein Gemisch von Sarcosin, Weizen-Aminosäuren und Palmitinsäure.

Ein bevorzugtes pflanzliches öl ist Jojobaöl, Galambutter, Avocado-öl, Avocadin, Calendula-öl und Aprikosenkern-öl, insbesondere Jojoba-öl.

Ein bevorzugter Emulgator ist Cetearyl glucoside, Methylglucose, Sesquistearat und Polyglycerol-3-methylglucosedistearat, insbesondere Cetearyl glucoside. Der Emulgator kann in einer Konzentration von etwa 1 bis 7 Gew-% vorhanden sein.

Cetearyl glucoside (CTFA-Name) ist eine wertvolle pflanzliche Komponente, deren Bestandteile Extraktionsprodukte sind. So ist die darin enthaltene Glucose aus Mais extrahiert und das Fett aus Kokosnußöl. Die Glucolipidstruktur ist frei von chemischen Verunreinigungen und üblichen Lösungsmitteln. Es wirkt hier sowohl emulgierend als auch feuchtigkeitsanlagernd.

Die bevorzugten waschaktiven Substanzen Decyl polyglucose und Acylglutamate (CTFA-Bezeichnungen) sind ebenfalls von Naturstoffen abgeleitete oberflächenaktive Mittel. Die waschaktive Substanz kann in einem Gehalt von etwa 1 bis 15 Gew-% vorhanden sein, vorzugsweise 3 - 10 Gew-%.

Die erfindungsgemäß eingesetzten Mikrokügelchen aus Polyoxymethylenharnstoff haben beispielsweise einen mittleren Durchmesser von 160 bis 200 *µ*m und liegen in Form eines weichen Pulvers mit einer leicht granulierten Textur vor. Sie weisen in ihrem Inneren einen Hohlraum auf, der mit dem pflanzlichen öl gefüllt ist, üben beim Verreiben auf der feuchten Haut eine milde Scheuer- bzw. Schleifwirkung aus und entfernen dadurch umfassend tote Hautzellen von der Haut. Gleichzeitig wird beim Auftragen und Verreiben der Hohlkörper geöffnet, und das darin enthaltene öl wird freigesetzt und kann seine feuchthaltende, pflegende und beruhigende Wirkung entfalten.

Als weitere Wirkstoffe können in dem erfindungsgemäßen Mittel Dexpanthenol, Hyaluronsäure, Phospholipide und Honigextrakt vorhanden sein.

Es wurde gefunden, daß durch das Zusammenwirken aller Komponenten der Formulierung nicht nur die angestrebte Reinigungswirkung auftritt, d.h. die Entfernung toter Hautzellen, sondern daß darüber hinaus ohne Porenverstopfung eine wesentlich verbesserte Weichheit der Haut festzustellen ist.

Ein überraschender Effekt besteht darin, daß eine außerordentlich gute Hydratationswirkung auf der Haut auftritt, die gegenüber Vergleichspräparaten um mehr als 100 % erhöht ist. Zu den üblichen kosmetischen Träger- und Hilfsstoffen, die weiterhin in der erfindungsgemäßen Zusammensetzung enthalten sein können, gehören Emulgatoren, verschiedene ölkomponenten (wie Fette, öle, Ester, Siliconöl usw.), weitere Stoffe wie Allantoin, Verdikkungsmittel, Konservierungsmittel, Parfümöle, Komplexbildner, aktive Substanzen wie z.B. Allantoin sowie Wasser.

Das erfindungsgemäße Hautreinigungsmittel wird vorteilhaft auf die feuchte Haut aufgetragen und mit kreisförmigen Bewegungen einige Minuten auf der Haut massiert. Anschließend wird es mit warmem Wasser abgespült. Mit einer auf diese Weise gereinigten Haut wird z.B. bei anschließendem Auftragen eines Selbstbräunungsmittels eine besonders gleichmäßige und natürliche Färbung der Haut erreicht. Eine solche Kombination von Reinigung und Bräunung liegt daher ebenfalls im Schutzbereich der Erfindung.

Durchgeführte Reiztests mit einer erfindungsgemäßen Hautreinigungszusammensetzung ergaben praktisch kein Reizungen, wodurch die vorteilhaft milde Wirkung des kosmetischen Präparates bestätigt wird.

Die Erfindung soll nachstehend durch Beispiele näher erläutert werden, die jedoch keine Einschränkung der Erfindung darstellen. Die Prozentangaben sind stets auf das Gewicht bezogen, sofern nicht anderes angegeben ist. Für die Inhaltsstoffe wurden teilweise die CTFA-Bezeichnungen verwendet.

### Beispiel 1

| Phase A | |
|---|---|
| Cetearyl glucoside | 5 % |
| Hexyllaurate | 7 % |
| Bienenwachs | 1 % |
| Isononyl Isononate | 8 % |
| Weizenproteine | 0,3 % |
| Vitamingemisch C+E | 0,1 % |

| Phase B | |
|---|---|
| deionisiertes Wasser | 42,1 % |
| D-Gluconsäure | 5 % |
| Propylenglycol | 2 % |
| Triethanolamin | 0,2 % |

| Phase C | |
|---|---|
| Laureth-7/Polyacrylamid/C13-14 Iosparaffin | 4,5 % |

| Phase D | |
|---|---|
| Konservierungsmittel | 0,8 % |

| Phase E | |
|---|---|
| Parfüm | 0,5 % |

| Phase F | |
|---|---|
| Decylpolyglucose | 7 % |

| Phase G | |
|---|---|
| deionisiertes Wasser | 1 % |
| Aloe vera Gel | 1 % |

| Phase H | |
|---|---|
| Polyethylen | 11 % |

| Phase I | |
|---|---|
| Polyoxymethylenharnstoff-Kapseln (3M brand PMU Capsules®) mit ca 65% Jojobaöl | 4 % |

Es wurden separat 8 Phasen hergestellt, die nacheinander miteinander vermischt wurden, teilweise unter intensiver Homogenisierung. Dabei wurden die Phasen A bis D bei erhöhter Temperatur (40 bis 70 °C) und die Phasen E bis I zum Schluß bei Umgebungstemperatur (unter 35 °C) eingearbeitet.

Man erhielt eine milde, cremige Emulsion mit reinigenden, glättenden und feuchtigkeitsspendenden Eigenschaften. Die Creme wurde auf die feuchte Haut aufgetragen und mit kreisförmigen Bewegungen massiert. Die milden (sanften) Schleifmittel (Peelingkörper) entfernten abgestorbenen Hautzellen und führten zu einer gleichmäßig weichen Erscheinungsform der Haut.

### Beispiel 2

| Phase A | |
|---|---|
| Cetearyl glucoside | 3 % |
| Hexyllaurate | 10 % |
| Bienenwachs | 3 % |
| Isononyl Isononanoat | 5 % |
| Weizenproteine | 0,2 % |
| Vitamingemisch C+E | 0,2 % |

| Phase B | |
|---|---|
| deionisiertes Wasser | 43,1 % |
| D-Gluconsäure | 3 % |
| Propylenglycol | 3 % |
| Triethanolamin | 0,2 % |

| Phase C | |
|---|---|
| Laureth-7/Polyacrylamid/C13-14 Iosparaffin 4,5 % | |

| Phase D | |
|---|---|
| Konservierungsmittel | 0,8 % |

| Phase E | |
|---|---|
| Parfüm | 0,5 % |

| Phase F | |
|---|---|
| Decylpolyglucose | 5 % |

| Phase G | |
|---|---|
| deionisiertes Wasser | 1 % |
| Aloe vera Gel | 1,5 % |

| Phase H | |
|---|---|
| Polyethylen | 11 % |

| Phase I | |
|---|---|
| Polyoxymethylenharnstoff-Kapseln | |
| mit ca 65% Jojobaöl | 5 % |

Es wurden separat 8 Phasen hergestellt, die nacheinander miteinander vermischt wurden, teilweise unter intensiver Homogenisierung. Dabei wurden die Phasen A bis D bei erhöhter Temperatur (40 bis 70 °C) und die Phasen E bis I zum Schluß bei Umgebungstemperatur (unter 35 °C) eingearbeitet.

Man erhielt eine Creme mit Eigenschaften wie im obigen Beispiel 1.

### Vergleichsbeispiel 1

Die erfindungsgemäße Creme gemäß Beispiel 1 (in der Tabelle "A") wurde einem Vergleichsversuch unterworfen mit einem Handelsprodukt (Oil of Olay, in der Tabelle "B"), das ebenfalls eine feuchtigkeitsfördernde Wirkung und eine Reinigungswirkung hatte.

Dabei wurden jeweils 2 mg des Präparates pro cm² Haut auf eine zuvor befeuchtete Hautzone aufgetragen, anschließend abgewaschen und abgetrocknet.

Die Messung erfolgte mit einem AFM Corneometer® .

In der nachfolgenden Tabelle 1 sind die Ergebnisse dieser

Messungen aufgeführt.

**Tabelle 1**

| Zeit | a) Mittelwert b) Streuung | Hydratationsverbesserung im Vergleich mit unbehandelter Stelle | |
|---|---|---|---|
| | | A | B |
| vorher | a) | 0,9% | 0,3% |
| | b) | 1,8 | 2,4 |
| unmittelbar | a) | 31,9% | 23,9% |
| danach | b) | 5,8 | 6,5 |
| nach 0,5h | a) | 10,2% | 3,2% |
| | b) | 2,5 | 1,3 |
| nach 1h | a) | 7,8% | 4,2% |
| | b) | 2,6 | 1,9 |
| nach 2h | a) | 5,7% | 2,1% |
| | b) | 2,0 | 1,8 |
| nach 3h | a) | 3,0% | 1,3% |
| | b) | 2,0 | 1,7 |

## Patentansprüche

1. Kosmetisches Hautreinigungsmittel auf Basis natürlicher Wirkstoffe, gekennzeichnet durch eine wäßrige, nicht-ölige Suspension, bestehend aus
(a) Mikrokügelchen eines Polyoxymethylenharnstoffes, die in ihrem Inneren ein flüssiges, natürliches, pflanzliches öl tragen,
(b) Mitteln auf natürlicher Basis zur Anlagerung von Wasser an die Haut, ausgewählt unter Aloe vera Gel, Jojoba-öl, Cetearyl glucoside, Lipacide PVB und Gemischen davon, sowie als weiteren Stoff Propylenglycol,
(c) einem oder mehreren natürlichen Emulgatoren,
(d) natürlichen waschaktiven Substanzen, und
(e) weiteren Hilfs- und Trägerstoffen.

2. Mittel nach Anspruch 1, dadurch gekennzeichnet, daß das natürliche pflanzliche öl Jojobaöl ist.

3. Mittel nach Anspruch 1, dadurch gekennzeichnet, daß der Emulgator Cetearyl glucoside ist.

4. Mittel nach Anspruch 1, dadurch gekennzeichnet, daß die natürliche waschaktive Substanz Decyl polyglucose ist.

5. Mittel nach Anspruch 1, dadurch gekennzeichnet, daß die Suspension Nährstoffe für die Haut enthält, wie Proteine, insbesondere Weizenproteine.

6. Mittel nach Anspruch 1, dadurch gekennzeichnet, daß die Suspension als weiter Hilfsstoffe Radikalfänger und/oder Antioxidationsmittel enthält, ausgewählt aus der Gruppe, die aus Vitaminen wie Vitamin C und E und Vitamingemischen besteht.

7. Mittel nach Anspruch 1, dadurch gekennzeichnet, daß die Suspension Erweichungsmittel enthält.

8. Mittel nach Anspruch 1, dadurch gekennzeichnet, daß die Suspension die Mikrokügelchen in einem Anteil von 0,5 bis 10 Gew.-% enthält, vorzugsweise 0,5 bis 5 Gew-%.

9. Mittel nach Anspruch 1, dadurch gekennzeichnet, daß die Suspension die Mittel zur Anlagerung von Wasser in einem Anteil von 9 bis 15 Gew.-% enthält, vorzugsweise 9 bis 11 Gew-%.

10. Mittel nach Anspruch 1, dadurch gekennzeichnet, daß der Emulgatoranteil in der Suspension 1 bis 7 Gew-% beträgt.

11. Mittel nach Anspruch 1, dadurch gekennzeichnet, daß die Suspension die waschaktiven Substanzen in einem Anteil von 1 bis 15 Gew.-% enthält.

## Claims

1. A cosmetic skin cleanser preparation based on natural active ingredients, comprising an aqueous non-oily suspension, comprising
(a) microspheres of a polyoxymethylene urea containing a natural liquid vegetable oil,
(b) agents based on natural substances for moisturizing the skin, selected from aloe vera gel, jojoba oil, cetaryl glucosides, lipacids PVB and mixtures thereof, plus propylene glycol as an additional ingredient,
(c) one or more natural emulsifiers,
(d) natural cleansing substances, and
(e) other excipients and vehicles.

2. A preparation according to Claim 1, wherein the natural vegetable oil is jojoba oil.

3. A preparation according to Claim 1, wherein the emulsifier is cetaryl glucoside.

4. A preparation according to Claim 1, wherein the natural cleansing substance is decyl polyglucose.

5. A preparation according to Claim 1, wherein the suspension contains nutrients for the skin, such as proteins, specifically wheat proteins.

6. A preparation according to Claim 1, wherein the suspension contains radical scavengers and/or antioxidants as additional additives selected from the group comprising vitamins such as vitamins C and E and vitamin mixtures.

7. A preparation according to Claim 1, wherein the suspension contains emollients.

8. A preparation according to Claim 1, wherein the suspension contains microspheres in the amount of 0.5 to 10 percent by weight, preferably 0.5 to 5 percent by weight.

9. A preparation according to Claim 1, wherein the suspension contains the moisturizing agents in an amount of 9 to 15 percent by weight, preferably 9 to 11 percent by weight.

10. A preparation according to Claim 1, wherein the emulsifier content in the suspension is 1 to 7 percent by weight.

11. A preparation according to Claim 1, wherein the suspension contains the cleansing substances in an amount of 1 to 15 percent by weight.

## Revendications

1. Produit cosmétique pour le nettoyage de la peau, à base de principes actifs naturels, caractérisé par une suspension aqueuse, non huileuse, composée :
(a) de microbilles faites d'une polyoxyméthylène urée et contenant une huile végétale naturelle liquide,
(b) d'agents à base naturelle pour fixer de l'eau sur la peau, choisis parmi des gels d'Aloe vera, l'huile de jojoba, le glucoside de cétéaryle, le Lipacide PVB et des mélanges de ceux-ci, ainsi que du propylène glycol en tant que substance supplémentaire,
(c) d'un ou plusieurs émulsifiants naturels,
(d) de substances naturelles ayant une action lavante, et
(e) d'autres adjuvants et véhicules.

2. Produit selon la revendication 1, caractérisé en ce que l'huile végétale naturelle est l'huile de jojoba.

3. Produit selon la revendication 1, caractérisé en ce que l'émulsifiant est le glucoside de cétéaryle.

4. Produit selon la revendication 1, caractérisé en ce que la substance naturelle ayant une action lavante est le décylpolyglucose.

5. Produit selon la revendication 1, caractérisé en ce que la suspension contient des substances nutritives pour la peau telles que des protéines, en particulier des protéines de blé.

6. Produit selon la revendication 1, caractérisé en ce que la suspension contient comme autres adjuvants des piégeurs de radicaux et/ou des antioxydants choisis dans le groupe comprenant des vitamines telles que les vitamines C et E et des mélanges de vitamines.

7. Produit selon la revendication 1, caractérisé en ce que la suspension contient des agents émollients.

8. Produit selon la revendication 1, caractérisé en ce que la suspension contient les microbilles en une proportion de 0,5 à 10% en poids, de préférence de 0,5 à 5% en poids.

9. Produit selon la revendication 1, caractérisé en ce que la suspension contient les agents de fixation d'eau en une proportion de 9 à 15% en poids, de préférence de 9 à 11% en poids.

10. Produit selon la revendication 1, caractérisé en ce que la proportion d'émulsifiant dans la suspension est de 1 à 7% en poids.

11. Produit selon la revendication 1, caractérisé en ce que la suspension contient les substances ayant une action lavante en une proportion de 1 à 15% en poids.
